# EUROPEAN PATENT SPECIFICATION

(11) **EP 2 216 324 B1**
(45) Date of publication and mention of the grant of the patent: **13.03.2013**
(21) Application number: 08877340.3
(22) Date of filing: 25.11.2008
(51) Int. Cl.: C07C 281/18, C07C 279/36, C07C 277/08, A01N 47/44

(54) **Condensed amino nitroguanidine compounds, synthesis and use as botanical insecticides thereof**
Kondensierte Aminonitroguanidinzusammensetzungen, Synthese und Verwendung davon als botanische Insektizide
Aminonitroguanidines condensées, leur synthèse et leur emploi en tant qu'insecticides botaniques

(43) Date of publication of application: 11.08.2010
(73) Proprietor: Qin, ZhaoHai, Beijing 100094 (CN)
(72) Inventor: QIN, ZhaoHai, Haidian District Beijing 100094 (CN); MA, Yongqiang, Haidian District Beijing 100094 (CN); SU, Wangcang, Haidian District Beijing 100094 (CN); WANG, Lei, Haidian District Beijing 100094 (CN); ZHANG, Zheng, Haidian District Beijing 100094 (CN); ZHAO, Bangbin, Haidian District Beijing 100094 (CN); FANG, Jiangsheng, Haidian District Beijing 100094 (CN)
(74) Representative: Graf von Stosch, Andreas
(86) International application number: PCT/CN2008/001919
(87) International publication number: WO 2010/060231

(56) References cited:
- EP-A1- 0 974 579
- WO-A1-93/04032
- RONALD A. HENRY ET AL.: "The Reaction of Methylamine with Nitroaminoguanidine", JOURNAL OF THE AMERICAN CHEMICAL SOCIETY., vol. 73, 1951, pages 1858-1859, XP002615575, USAMERICAN CHEMICAL SOCIETY, WASHINGTON, DC. ISSN: 0002-7863

## Description

### Field of the Invention

The present invention relates to hydrocarbylidene nitrohydrozinecarboximidamides and a method for making the same, as well as their uses as an insecticide.

### Background of the Invention

Nicotine is a natural alkaloid, which has been used as an insecticide as early as the nineteen century, and the target which acts on is the postsynaptic nicotinic acetylcholine receptor (nAchRs). However, the insecticidal activity of Nicotine is low, and it is highly toxic to human beings. With the Nicotine being as a leading compound, such insecticides have been fully developed and successive several generations of products have been commercially developed, since Bayer AG successfully developed the neonicotiniod insecticides, Imidacloprid, in 1980s. Neonicotine insecticides have high insecticidal activities and broad insecticidal spectrum, and are safe to mammalian and other environmental lives. The above insecticides have structural general formula as shown in formula A.

In contrast, the use of semicarbazone compounds as insecticides is developed a little late, however, the development of such insecticides has become a new hotspot since Dupont Corporation successfully developed indoxacarb in 1992. The action target of such insecticides is sodium ion channel, and they specially effect on nearly all of Lepidoptera pest, but they are safe to each of mammalian, avian and aquatic animal. Such insecticides have the structural general formula as shown in Formula B.

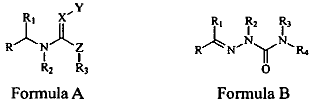

As to the biocompatibility, insecticides acting on a single target generally quickly result in resistance, and insecticides acting on multiple targets do slowly.

### Summary of the Invention

The object of the present invention is to provide hydrocarbylidene nitrohydrozine carboximidamides and a method for making the same.

EP 0 974 579 relates to a specific nitroisourea derivative, which is an important intermediate for the production of nitroguanidine derivative having insecticidal activity. In addition, EP 0974 579 discloses a process for producing the nitroisourea derivative and a process for producing the nitroguanidine derivative using the nitroisourea derivative.

The present invention provides hydrocarbylidene nitrohydrozinecarboximidamides , which has structural general formula as shown in formula I:

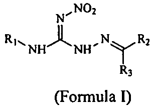

Wherein:
R1 is C1 - C10 saturated and/or unsaturated aliphatic hydrocarbonyl, benzyl, substituted benzyl, halogenated picolyl, halogenated thiazolyl methyl, tetrahydrofuryl methyl or oxazolyl methyl, wherein the substituent of said substituted benzyl can be halogen (specificially F, Cl, Br, and I), amino, hydroxy, C1 - C5 alkyl or C1 - C5 alkoxyl , and the like;
R2 is hydrogen, C1 - C5 saturated and/or unsaturated aliphatic hydrocarbonyl, substituted phenyl, pyridyl or substituted pyridyl, wherein the substituent of said substituted phenyl can be halogen, hydroxy, amino, C1 - C5 alkyl, C1 - C5 alkoxyl, aryl (such as phenyl, pyridyl, imidazolyl, oxazolyl, and thiazyl), aryloxy (such as phenoxy, and pyridinyloxy) and the like, and the substituent of said substituted pyridyl can be halogen, C1 - C5 alkyl, C1 - C5 alkoxyl, aryloxy (such as phenoxy, and pyridinyloxy) and the like;
R3 is hydrogen, C1 - C10 saturated and/or unsaturated aliphatic hydrocarbonyl, furyl, phenyl, substituted phenyl, benzyl or substituted benzyl, wherein the substituent of said substituted phenyl can be halogen, hydroxy, amino, substituted amino (such as methylamino, and dimethylamino), nitro, C1 - C5 alkyl, C1 - C5 alkoxyl, aryl (such as phenyl, pyridyl, imidazolyl, oxazolyl, thiazyl), aryloxy (such as phenoxy and pyridinyloxy) and the like, and the substituent of said substituted benzyl can be halogen, hydroxy, amino, C1 - C5 alkyl, C1 - C5 alkoxyl, aryl (such as phenyl, pyridyl, imidazolyl, oxazolyl, and thiazyl), aryloxy (such as phenoxy and pyridinyloxy), and the like;

Preferred R1 is C1 - C10 unsaturated aliphatic hydrocarbonyl, halogenated picolyl, halogenated thiazolyl methyl or tetrahydrofuryl methyl; preferred R2 is hydrogen or C1 - C5 saturated and/or unsaturated aliphatic hydrocarbonyl; and preferred R3 is substituted phenyl or C1 - C10 saturated and/or unsaturated aliphatic hydrocarbonyl;

More preferred R1 is allyl, propargyl or chloro-picolyl; more preferred R2 is hydrogen; and more preferred R3 is substituted phenyl or C3 - C7 saturated and/or unsaturated aliphatic hydrocarbonyl.

The saturated and/or unsaturated aliphatic hydrocarbonyl of the present invention can be linear or branched.

The present invention provides a method for making the hydrocarbylidene nitrohydrozinecarboximidamides shown in structural general formula I, including the following steps:
1) Reacting nitroguanidine with hydrazine hydrate to form N'-nitrohydrazinecarboximidamide shown by formula II; 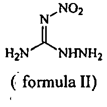
2) Reacting N'-nitrohydrazinecarboximidamide shown by formula II with carbonyl compounds shown by the structural general formula III under acid catalysis, to form hydrocarbylidene nitrohydrozinecarboximidamides shown by the structural general formula IV: 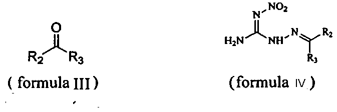
3) Reacting the compounds shown by the structural general formula IV with compounds shown by the structural general formula V (halohydrocarbons or sulfonic esters) under alkali catalysis, to form compounds shown by the structural general formula I;

   R1-X formula V

Wherein, R2 of formula II and formula III are hydrogen, C1 - C5 saturated and/or unsaturated aliphatic hydrocarbonyl, phenyl, substituted phenyl, pyridyl or substituted pyridyl, wherein the substituent of said substituted phenyl can be halogen, hydroxy, amino, C1 - C5 alkyl, C1 - C5 alkoxyl, aryl (such as phenyl, pyridyl, imidazolyl, oxazolyl, and thiazyl), aryloxy (such as phenoxy, and pyridinyloxy) and the like, and the substituent of said substituted pyridyl can be halogen, C1 - C5 alkyl, C1 - C5 alkoxyl, aryloxy (such as phenoxy, and pyridinyloxy), and the like;
R3 is hydrogen, C1 - C10 saturated and/or unsaturated aliphatic hydrocarbonyl, furyl, phenyl, substituted phenyl, benzyl or substituted benzyl, wherein the substituent of said substituted phenyl can be halogen, hydroxy, amino, substituted amino (such as methylamino, and dimethylamino), C1 - C5 alkyl, C1 - C5 alkoxyl, aryl (such as phenyl, pyridyl, imidazolyl, oxazolyl, and thiazyl), aryloxy (such as phenoxy, and pyridinyloxy) and the like, and the substituent of said substituted benzyl can be halogen, hydroxy, amino, C1 - C5 alkyl, C1 - C5 alkoxyl, aryl (such as phenyl, pyridyl, imidazolyl, oxazolyl, and thiazyl), aryloxy (such as phenoxy, and pyridinyloxy), and the like;
R1 of formula V is C1 - C10 saturated and/or unsaturated aliphatic hydrocarbonyl, benzyl, substituted benzyl, halogenated picolyl, halogenated thiazolyl methyl, tetrahydrofuryl methyl or oxazolyl methyl, wherein the substituent of said substituted benzyl can be halogen, amino, hydroxy, C1 - C5 alkyl or C1 - C5 alkoxyl and the like, and X of formula V is C1, Br, I, OTos (p-tosyloxy) or OTf (trifluoromethane sulfonyl);

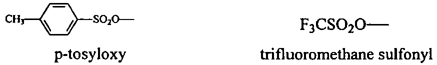

Preferred R1 is C1 - C10 unsaturated aliphatic hydrocarbonyl, halogenated picolyl, halogenated thiazolyl methyl or tetrahydrofuryl methyl, preferred R2 is hydrogen or C1 - C5 saturated and/or unsaturated aliphatic hydrocarbonyl; preffered R3 is substituted phenyl or C1 - C10 saturated and/or unsaturated aliphatic hydrocarbonyl;

More preferred R1 is allyl, propargyl or chloro-picolyl, more preferred R2 is hydrogen, more preferred R3 is substituted phenyl or C3-C7 saturated and/or unsaturated aliphatic hydrocarbonyl.

The reaction in step 1) is conducted in a solvent, and said solvent can be water; and the reaction temperature of said reaction is 45-70°C. The molar ratio of nitroguanidine to hydrazine hydrate in step 1) is 1:1-1:1.5.

The reaction in step 2) is conducted in a solvent, and said solvent can be anhydrous ethanol or methanol; the reaction temperature of said reaction can be 50-80°C; and the acids used in said reaction can be acetic acid or p-toluenesulfonic acid. The molar ratio of N'-nitrohydrazinecarboximidamide shown by formula II to carbonyl compounds shown by the structural general formula III in step 2) is 1:1-1:2.

The reaction in step 3) is conducted in a solvent, and said solvent can be DMF (dimethylformamide) or DME (dimethylacetamide); the reaction temperature of said reaction can be 0-50°C and the alkalies used in said reaction can be sodium hydride, sodium ethoxide, sodium methoxide or sodium amide. The molar ratio of the compounds shown by the structural general formula IV to the compounds shown by the structural general formula V in step 3) is (1:1.2)-(1:2.5).

Another object of the present invention is to provide the use of hydrocarbylidene nitrohydrozinecarboximidamides shown by the structural general formula I.

The present invention provides the use of hydrocarbylidene nitrohydrozinecarboximidamides shown by the structural general formula I, wherein the compounds shown by the structural general formula I or pharmaceutically acceptable salts thereof or pharmaceutical compositions containing any of them can be used to prepare plant insecticides.

A further object of the present invention is to provide a plant insecticidal drug or formulation.

The active ingredient of plant insecticidal drugs or formulations provided by the present invention is hydrocarbylidene nitrohydrozinecarboximidamides shown by the structural general formula I or pharmaceutically acceptable salts thereof.

The mass percent content of the active ingredient of said insecticidal drugs or formulations is 0.01 %-99.99%.

Said insecticides can be processed into any acceptable dosage form as required. For example, the dosage form can be suspension, emulsion, aerosol, wettable powder, emulsifiable concentrate, and granule.

The preparation methods of dosage form are exemplified as follows:

The preparation of the suspension: the content of active ingredient in the conventional formulation is 5% - 35%. With the water being media, active pharmaceutical ingredients, water dispersant, suspending agent and antifreeze agent and the like, are added into the sander and are ground, to prepare suspension.

The preparation of the wettable powder: according to the requirement of formulation, active pharmaceutical ingredients, a variety of surfactants and solid diluents, and the like, are fully mixed, and after ultrafinly grinding, the wettable powder product of predetermined content can be obtained. In order to prepare the wettable powder suitable for spraying, active pharmaceutical ingredients and comminuted solid powder such as clay, inorganic silicate, carbonate and wetting agent, adhesive and/or dispersant can also constitute a mixture.

The preparation of the emulsifiable concentrate: according to the requirement of formulation, the active ingredients are dissolved into organic solvents, and emulsifying agents and other adjuvants are added and processed to form the formulation. Solvents can be toluene, xylene, methanol, and the like, and a cosolvent if necessary; and the other adjuvants including stabilizer, permeate agent and erosion inhibitor, and the like.

The compounds shown by the structural general formula I provided by the present invention and the insecticidal drugs or formulations with said compounds being active ingredient can control and kill a broad range of pests, which includes sucking insects , biting insects and other plant pests, pests of grain storage, and sanitary pests causing health hazard, and the like.

The pests are exemplified as follows:

Homoptera pests include aphididae, aleyrodidae, delphacidae, psyllidae, jassidae and coccidae pests. Aphididae pests can specifically be *Aphis gossypii, bean aphid, Myzus. persicae, Hyalopterus pruni, turnip aphid* or *cabbage aphid*; delphacidae pests can specifically be *Nilaparvata lugens* or *rice planthopper;* aleyrodidae pests can specifically be *Bemisia tabaci gennadius*; jassidae pests can specifically be *rice leafhopper**(Nephotettix bipunctatus* ); and coccidae pests can specifically be *arrowhead scale(Unaspis yanonensis).*

Lepidoptera pests include noctuidae and plutellidae pests. Noctuidae pests can specifically be *asparagus caterpillar, Spodoptera litura Fab* or *Helicoverpa armigera;* plutellidae pests can specifically be *Plutella xylostella*.

The plant insecticidal drugs or formulations provided by the present invention can be used to prevent insect pests of plants. Especially, the plant insecticidal drugs or formulations have special efficiency against piercing-sucking type pests, scratching type mouthparts pests, such as various aphid, plant hopper, leafhopper, mealworm and *Thrips palmi,* and have high efficiency against *Helicoverpa armigera* and *asparagus caterpillar*.

A further object of the present invention is to provide a method for preventing insect pests of plants.

The method for preventing from insect pests of plants provided by the present invention is to apply the plant insecticidal drugs or formulations provided by the present invention to plant leaves and/or plant fruits and/or plant seeds, and the places where the plant leaves and/or plant fruits and/or plant seeds are growing or are expected to be grown. The active ingredient of the plant insecticidal drugs or formulations is administrated at the concentration of 1-600mg/L, and preferably, at the concentration of 3-50 mg/L.

### The best modes of carrying out the invention

Hydrocarbylidene nitrohydrozinecarboximidamides of the present invention can be synthesized by following steps:

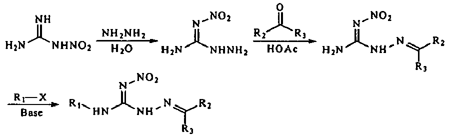

The present invention is further illustrated in combination with the following specific examples. It should be understood that these examples are used only to illustrate the present invention, but not to limit the scope of the present invention. In the following examples, experimental methods which do not indicate particular conditions usually are carried out under normal conditions or under the conditions proposed by the manufacturer. Unless otherwise specified, percentage and parts are calculated based on the mass.

In the following, using 2-isobutylidene-N'-nitrohydrozinecarboximidamide and 2-(2'-nitrophenyl methylene)-N-nitrohydrozinecarboximidamide as examples, the preparation method of hydrocarbylidene nitrohydrozinecarboximidamides provided by formula (I) of the present invention is illustrated.

### Example 1

The synthesis of 2-isobutylidene-N'-nitrohydrozinecarboximidamide (the compound I of formula I, wherein R1 is methyl, R2 is hydrogen, and R3 is isopropyl):

### (1) the synthesis of N'-nitrohydrozinecarboximidamide

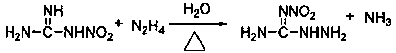

To 250mL three-necked flask, 5.0g (0.048mol) nitroguanidine and 70mL water were sequentially added. It was heated to 55°C under magnetic stirring, and the aqueous solution of 85% by mass of hydrazine hydrates (wherein, the mass of the hydrazine hydrates added was 3.5g (0.059mol)) was slowly added dropwise through a drop funnel. The reaction was continued for 20 minutes, while the temperature of materials was kept between 55 and 60°C. When the materials turned into an orange clear liquid, it was cooled quickly with an ice water bath, and about 6mL concentrated HCl (the mass percent is 37%) was slowly added dropwise to adjust pH as 5 - 6; the materials were continued to be cooled to 2 - 3°C and lasted for 1 hour. The resulting product was filtered under reduced pressure, and washed with a little ice water, and air-dried in a fume hood. The resulting product was recrystallized with hot water, and 2.74g light yellow powder (N'-nitro amino guanidine) was obtained in 48% yield, and the melting point of which is 191 - 192°C.

Structural characteristic data are provided as follows:
¹H NMR(DMSO-d₆ , δppm) : 4.69 (s, 2H, -NHNH₂), 7.56 (s, 1H,-NHNH₂), 8.27 (s, 1H,-NHNO₂), 9.33 (s, 1H, C=NH).

### (2) The synthesis of 2-isobutylidene-N'-nitrohydrozinecarboximidamide

To 250mL three-necked flask, 24g (0.2mol) N'-nitrohydrozinecarboximidamide, 100mL anhydrous ethanol, and 2.4mL glacial acetic acid were sequentially added. It was heated to 65°C under magnetic stirring, and 19.0g (0.24mol) isobutylaldehyde (the compounds in formula III, wherein R2 is hydrogen, and R3 is isopropyl) was slowly added dropwise through a drop funnel. After the addition was complete, the mixture was heated to reflux, and the reaction was refluxed for 3 hours. The temperature was lowered, and the solvent was removed under the reduced pressure. The resulting crude product was recrystallized with ethanol-petroleum ether (3:1 by volume) to obtain 20g light yellow powder (2-isobutylidene-N'-nitrohydrozinecarboximidamide) in 52% yield, and the melting point of which is 76-78°C.

### (3) The synthesis of 2-isobutylidene-N-methyl-N'-nitrohydrozinecarboximidamide (compound 1)

To 50mL three-necked flask, 7.0g (0.04 mol) 2-isobutylidene-N'-nitrohydrozinecarboximidamides and 30mL anhydrous DMF were sequentially added. A drying tube was installed, magnetically stirring was run, and ice water bath was used to lower the temperature. The temperature was lowered to below 10°C, the solution of 70% by mass of sodium hydride in DMF was added in three times (wherein, the mass of added sodium hydride is 2.4g (0.07mol)), and the mixture was reacted for 1 hour. The solution of 11.5g (0.08mol) iodomethane and 30mL anhydrous DMF was slowly added dropwise through a drop funnel. After the addition was complete, the ice water bath was removed, and the temperature was raised to room temperature naturally. After the mixture was reacted for 2 hours at room temperature, 150mL water was added, and solids were precipitated. The resulting product was seted, filtered, washed with water, and dried to obtain 2.9g colorless plate-like crystal (2-isobutylidene-N-methyl-N'-nitrohydrozinecarboximidamides) in 45% yield, which was recrystallized with ethanol-petroleum ether (1:2 by volume), and its melting point is 60-61°C .

Structural characteristic data are provided as follows:
¹HNMR(CDCl_{3,}δppm): 1.18-1.16(q, 6H) , 2.62-2.73(m, 1H), 3.36(d, 3H), 7.15-7.16(t, 1H), 7.45(s,1H), 9.05(s, 1H)

| elemental analysis | | | |
|---|---|---|---|
| | C% | H% | N% |
| theoretical value: | 38.50 | 7.00 | 37.41 |
| measured value : | 38.53 | 6.89 | 37.35 |

### Example 2

The synthesis of 2-(2'-nitrobenzylidene)-N-propyl-N'-nitrohydrozinecarboximidamides (the compound ZNQ-103 in formula I, wherein R1 is propyl, R2 is hydrogen, and R3 is o-nitrophenyl)

### (1) The synthesis of 2-(2'-nitrobenzylidene)-N'-nitrohydrozinecarboximidamides

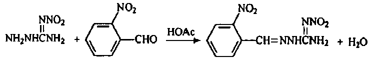

To 250mL three-necked flask, 2.0g (0.017mol) N'-nitrohydrozinecarboximidamide, 100mL anhydrous ethanol and 0.2mL glacial acetic acid were sequentially added. It was heated to 65°C under magnetic stirring, and the solution of 3.02g (0.020mol) o-nitrobenzaldehyde (the compounds in formula III, wherein R2 is hydrogen, and R3 is o-nitrophenyl) and 10mL anhydrous ethanol was slowly added dropwise through a drop funnel. After the addition was complete, the mixture was heated to reflux, and refluxed for 3 hours. The temperature was lowered, the solvent was removed under the reduced pressure, and solids were precipitated. The resulting crude product was recrystallized with chloroform to obtain 3.21g orange powder (2-(2'-nitrobenzylidene) -N'-nitrohydrozinecarboximidamide) in 75% yield, and the melting point is 225 - 226°C.

### (2) The synthesis of 2-(2'-nitrobenzylidene)-N-propyl-N'-nitrohydrozinecarboximidamides (ZNQ-103)

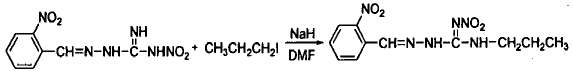

To 250mL three-necked flask, 2.0g (0.008mol) 2-(2'-nitrobenzylidene)-N'-nitrohydrozinecarboximidamides and 50mL anhydrous DMF were sequentially added. A drying tube was installed, magnetical stirring was run, and ice water bath was used to lower the temperature. The temperature was lowered to below 10°C, the solution of 70% by mass of sodium hydride in DMF was added in three times (wherein, the mass of added sodium hydride is 0.48g (0.014mol)), and the mixture was reacted for 1 hour. The solution of 2.72g (0.016mol) iodopropane and 10mL anhydrous DMF was slowly added dropwise through a drop funnel. After the addition was complete, the ice water bath was removed, and the temperature was raised to room temperature naturally. After the mixture was reacted for 2 hours at room temperature, 150mL water was added, and solids were precipitated. The resulting product was seted, filtered, washed with water, and dried to obtain 1.34g yellow powder (2-(2'-nitrobenzylidene)-N-propyl-N'-nitrohydrozinecarboximidamides) in 57% yield, which was recrystallized with ethyl acetate with 158°C melting point.

Strutral characteristic data are provided as follows:
¹H NMR (DMSO-d₆ , δppm): 3.89 (s, 3H, -CH₃), 4.79-4.82 (m, 2H, -CH₂-), 5.12-5.29 (m,2H, -NCH₂), 6.94-7.04 (m, 2H, -ArH), 7.39-7.45 (m, 1H, -ArH), 7.80-7.83 (m, 1H, -ArH), 8.29 (s, 1H, -CH=N-), 9.16 (s, 2H, -NH-×2).

| | | | |
|---|---|---|---|
| elemental analysis | | | |

| | C% | H% | N% |
|---|---|---|---|
| theoretical value: | 44.90 | 4.80 | 28.56 |
| measured value: | 44.90 | 4.73 | 28.51 |

### Example 3

### Test on the insecticidal activity of compounds of the present invention

Aphis, which belongs to Homoptera and has a piercing-sucking mouthpart, is a common pest for agricultural plant. The test subjects are *Myzus persicae, Hyalopterus pruni,* and *Aphis gossypii*, and the test is performed by the way of immersing. *Myzus persicae* were derived from cabbage fields in Hai Dian district, Beijing, *Halopterus pruni* were derived from peach tree in Dian district, Beijing, and *Aphis gossypii* were derived from hibiscus trees in Hai Dian district,Beijing. Each test was carried out using 3 day-old nymphae.

Operational precedure: 20 mg compounds provided by the present invention (caculating based on 100% content) was exactly weighed and formulated into 0.5% by mass of stock solution with 4mL acetone. Then, the stock solution was formulated into a series of liquid medicine to be determined using aqueous solution containing 0.1% by mass of Triton X-100. The leaves with aphides were chosen, and 3-day-old nymphae were left. After the leaves with aphides were immersed into the liquid medicine for 5 seconds and air-dried, the amount of aphides was recorded, and aphides were put into the culture dishes with moistened filter paper, then the culture dishes were capped and put into light incubator at (25±1)°C Each of medicament treated 30 or more aphides, while the blank controls being set up. After 5 - 48 hours, the results were examined.

The criteria for death judgment is: slightly touching the bodies of the pest, the one which can not normally creep is considered as dead individual.

Corrected mortality (%)=(mortality of samples-mortality of blank controls)/(1-mortality of blank controls)× 100%.

Aleyrodids belong to homoptera pests and has a piercing-sucking mouthpart. *Bemisia tabaci gennadius* was tested by the way of spraying.

Operational procedure: firstly, the samples were dissolved with dimethyl sulfoxide, then were formulated into a solution at concentration of 500mg/L using distilled water solution containing 0.01% by mass of Triton, and clean water was used as a blank control. The leaf of cotton was drilled using a punch to generate a leaf discs with a diameter of 18mm, and the drilled leaf disc was immersed into liquid medicine for 5 seconds, then air-dired at room temperature. Following about 2 hours, the leaf disc was placed back upwards on the bottom of digitiform tube plated with agar (the concentration is 1.4%). The treated digitiform tube was inversely placed on top of *Bemisia tabaci gennadius* (eclosion was carried out for 24 hours), and then leaf was flicked, *Bemisia tabaci gennadius* can automatically fly into the tube. Each tube can collect 25 bemisia tabaci gennadius. The tube orifice was packed (wrapped) with gauze, and then the tube was inversely placed in an insectarium for normal breeding. The breeding conditions are: L/D (photoperiod) is 14:10, T (temperature) is 26±2°C, RH (relative humidity) is 75±5%. After 1 hour, the condition of the test-insect was examined. If a test-insect was dead, then the test insect was not counted. After 48 hours, the results were collected.

*Helicoverpa armigera* belongs to noctuidae of Lepidoptera and has a chewing mouthpart. The compound sample was weighted using a balance with 0.0001 accuracy, and was formulated into a stock solution with Dimethylformamide (DMF). Then, the stock solution was formulated into a liquid medicine to be determined using aqueous solution containing 0.1 % by mass of Triton X-100.

Operational precedure: the cotton leaves were rinsed, and clean amaranth leaves were drilled using a punch to generate a leaf disc of 2 cm diameter, then the leaf disc was immersed into the liquid medicine for 10 seconds. After air-dried, the leaf disc was placed into ten-well test box. 2-day-old larvae of asparagus caterpillar were inoculated, one larva for each well, and the plastic wrap was covered. After being capped, the test box was placed into a light incubator at the temperature of (27+1) °C. After 48 hours, the results were examined. The individuals which body response abnormally or irresponsively to slightly touching with a hand setting (dial needle) were deemed as dead.

*Asparagus caterpillar* belongs to noctuidae of Lepidoptera and has a chewing mouthpart. The liquid medicine was formulated using medicament: the compound sample was weighted using a balance with 0.0001 accuracy, and formulated into stock solution with dimethylformamide (DMF). Then, the stock solution was formulated into the liquid medicine to be determined using aqueous solution containing 0.1% by mass of Triton X-100.

Operational precedure: the amaranth leaves were rinsed, and clean amaranth leaves were drilled using a punch to generate a leaf disc of 2 cm diameter, then the leaf disc was immersed into the liquid medicine for 10 seconds. After air-dried, the leaf disc was placed into ten-well test box. 2-day-old larvae of asparagus caterpillar were inoculated; one larva for each well, and the plastic wrap was covered. After being capped, the test box was placed into a light incubator at the temperature of (27+1) °C. After 48 hours, the results were examined. The individuals which body response abnormally or irresponsively to slightly touching with a hand setting (dial needle) were deemed as dead.

The results were shown in Table 1-4 below.

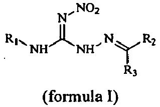

**Table 1. The results of mortality of compounds of formula I to various pests.**

| | | | | mortality(%) | | | |
|---|---|---|---|---|---|---|---|
| No. | R₁ | R₂ | R₃ | *Myzus persicae* (600µg/mL) | *Bemisia tabaci gennadi* us (500µg/ mL) | *Helicove rpa armigera* (500µg/ mL) | *asparagus caterpillar* (500µg/mL ) |
| 1 | CH₃ | H | CH(CH₃)₂ | 4.7 | | 40 | 40 |
| 2 | CH₂CH₃ | H | CH(CH₃)₂ | 53.6 | | 60 | 50 |
| 3 | CH₂CH₂CH₃ | H | CH(CH₃)₂ | 14.4 | | 30 | 20 |
| 4 | (CH₂)₃CH₃ | H | CH(CH₃)₂ | 31.6 | | 10 | 20 |
| 5 | CH₂CH=CH₂ | H | CH(CH₃)₂ | 27.8 | | 90 | 30 |
| 6 | CH₂C≡CH | H | CH(CH₃)₂ | 37.9 | | 100 | 60 |
| 7 | CH₂Ph | H | CH(CH₃)₂ | 18.4 | | 40 | 50 |
| 8 | 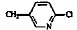 | H | CH(CH₃)₂ | 87.0 | | 60 | 40 |
| 9 | CH₂CH₃ | H | CH₂CH₃ | 54.2 | | 60 | 20 |
| 10 | CH₂CH₂CH₃ | H | CH₂CH₃ | 78.3 | | 80 | 20 |
| 11 | (CH₂)₃CH₃ | H | CH₂CH₃ | 1.5 | | 50 | 50 |
| 12 | CH₂CH=CH₂ | H | CH₂CH₃ | / | | 30 | 40 |
| 13 | CH₂C≡CH | H | CH₂CH₃ | 5.0 | | 50 | 0 |
| 14 | CH₂Ph | H | CH₂CH₃ | 18.8 | | 40 | 30 |
| 15 | 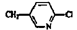 | H | CH₂CH₃ | / | | 10 | 20 |
| 16 | CH₃ | H | 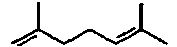 | 31.4 | | 30 | 70 |
| 17 | CH₂CH₃ | H | 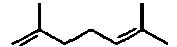 | 19.8 | | 40 | 40 |
| 18 | CH₂CH₂CH₃ | H | 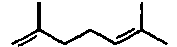 | / | | 50 | 60 |
| 19 | (CH₂)₃CH₃ | H | 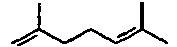 | 67.5 | | 40 | 60 |
| 20 | CH₂CH=CH₂ | H | 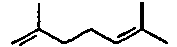 | 5.2 | | 0 | 50 |
| 21 | CH₂C≡CH | H | 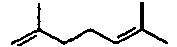 | 12.8 | | 20 | 30 |
| 22 | CH₂Ph | H | 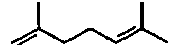 | 7.1 | | 20 | 20 |
| 23 | 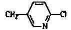 | H | 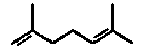 | 19.8 | | 30 | 40 |
| 24 | CH₂CH₃ | H | CH₂CH₂C H₃ | 4.7 | | 40 | 0 |
| 25 | CH₂CH₂CH₃ | H | CH₂CH₂C H₃ | / | | 50 | 10 |
| 26 | (CH₂)₃CH₃ | H | CH₂CH₂C H₃ | 65.3 | | 20 | 60 |
| 27 | CH₂CH=CH₂ | H | CH₂CH₂C H₃ | 65.1 | | 30 | 40 |
| 28 | CH₂C≡CH | H | CH₂CH₂CH₃ | 11.4 | | 60 | 70 |
| 29 | CH₂Ph | H | CH₂CH₂C H₃ | / | | 60 | 50 |
| 30 | 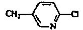 | H | CH₂CH₂C H₃ | 80.7 | | 40 | 40 |
| 31 | CH₃ | H | CH=CHCH₃ | 42.6 | | 60 | 10 |
| 32 | CH₂CH₃ | H | CH=CHCH₃ | 48.0 | | 0 | 0 |
| 33 | CH₂CH₂CH₃ | H | CH=CHCH₃ | 19.5 | | 70 | 70 |
| 34 | (CH₂)₃CH₃ | H | CH=CHCH₃ | / | | 40 | 60 |
| 35 | CH₂CH=CH₂ | H | CH=CHCH₃ | 56.7 | | 20 | 60 |
| 36 | CH₂C≡CH | H | CH=CHCH₃ | 35.0 | | 30 | 50 |
| 37 | CH₂Ph | H | CH=CHCH₃ | / | | 70 | 20 |
| 38 | 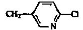 | H | CH=CHCH₃ | 1.2 | | 30 | 0 |
| 39 | CH₂CH₃ | H | CH=CH₂ | / | | 20 | 40 |
| 40 | CH₂CH₂CH₃ | H | CH=CH₂ | 8.3 | | 30 | 20 |
| 41 | (CH₂)₃CH₃ | H | CH=CH₂ | 74.7 | | 20 | 10 |
| 42 | CH₂CH=CH₂ | H | CH=CH₂ | 37.1 | | 20 | 0 |
| 43 | CH₂C≡CH | H | CH=CH₂ | / | | 40 | 20 |
| 44 | CH₂Ph | H | CH=CH₂ | 45.8 | | 30 | 0 |
| 45 | CH₂CH₂CH₃ | H | (CH₂)₅CH₃ | 16.9 | | 20 | 20 |
| 46 | (CH2)3CH3 | H | (CH₂)₅CH₃ | / | | 20 | 30 |
| 47 | CH₂CH=CH₂ | H | (CH₂)₅CH₃ | 23.7 | | 0 | 0 |
| 48 | CH₂C≡CH | H | (CH₂)₅CH₃ | 13.0 | | 30 | 0 |
| 49 | CH₂Ph | H | (CH₂)₅CH₃ | 28.6 | | 50 | 0 |
| 50 | 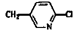 | H | (CH₂)₅CH₃ | 100 | | 20 | 0 |
| 51 | CH₂CH₂CH₃ | H | (CH₂)₃CH₃ | 13.3 | | 30 | 10 |
| 52 | (CH₂)₃CH₃ | H | (CH₂)₃CH₃ | 81.0 | | 20 | 60 |
| 53 | CH₂CH=CH₂ | H | (CH₂)₃CH₃ | 81.0 | | 40 | 80 |
| 54 | CH₂C≡CH | H | (CH₂)₃CH₃ | 47.6 | | 40 | 10 |
| 55 | CH₂Ph | H | (CH₂)₃CH₃ | 49.2 | | 10 | 30 |
| 56 | 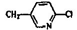 | H | (CH₂)₃CH₃ | 100 | | 70 | 20 |
| 57 | CH₃ | H | 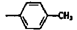 | 4.3 | | 20 | 20 |
| 58 | CH₂CH_{3.} | H | 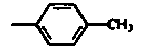 | 86.0 | | 30 | 30 |
| 59 | CH₂CH₂CH₃ | H | 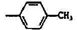 | / | | 50 | 0 |
| 60 | (CH₂)₃CH₃ | H | 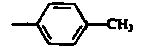 | 93.2 | | 0 | 0 |
| 61 | CH₂CH=CH₂ | H | 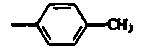 | 82.4 | | 80 | 80 |
| 62 | CH₂C≡CH | H | 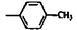 | 8.1 | | 60 | 60 |
| 63 | CH₂CH₃ | H | 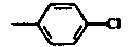 | / | | 10 | 100 |
| 64 | CH₂CH₂CH₃ | H | 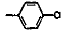 | 35.6 | | 50 | 50 |
| 65 | (CH₂)₃CH₃ | H | 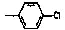 | 1.5 | | 0 | 60 |
| 66 | CH₂Ph | H | 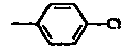 | 33.2 | | 10 | 70 |
| 67 | CH₂CH=CH₂ | H | 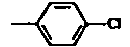 | / | | 10 | 50 |
| 68 | CH₂C≡CH | H | 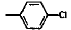 | 37.4 | | 0 | 70 |
| 69 | CH₂CH₃ | H | 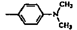 | 90.6 | | 30 | 80 |
| 70 | CH₂CH₂CH₃ | H | 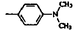 | / | | 40 | 80 |
| 71 | CH₂Ph | H | 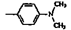 | / | | 30 | 90 |
| 72 | CH₂CH=CH₂ | H | 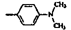 | 23.6 | | 50 | 50 |
| 73 | CH₂CH₂CH₃ | H | 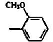 | 20.2 | | 50 | 70 |
| 74 | (CH₂)₃CH₃ | H | 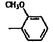 | 40.4 | | 0 | 30 |
| 75 | CH₂Ph | H | 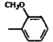 | 92.8 | | 30 | 90 |
| 76 | CH₂CH=CH₂ | H | 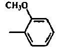 | 3.4 | | 40 | 80 |
| 77 | CH₂C≡CH | H | 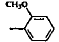 | 4.2 | | 30 | 50 |
| 78 | CH₃ | H | 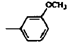 | 48.3 | | 80 | 60 |
| 79 | CH₂CH₃ | H | 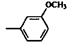 | 17.3 | | 0 | 60 |
| 80 | CH₂CH₂CH₃ | H | 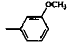 | / | | 50 | 70 |
| 81 | (CH₂)₃CH₃ | H | 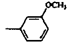 | 65.2 | | 40 | 60 |
| 82 | CH₂Ph | H | 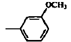 | / | | 60 | 50 |
| 83 | CH₂CH=CH₂ | H | 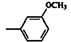 | / | | 20 | 50 |
| 84 | CH₂C≡CH | H | 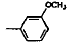 | 35.0 | | 10 | 70 |
| 85 | CH₂CH₃ | H | 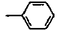 | 51.5 | | 20 | 40 |
| 86 | CH₂CH₂CH₃ | H | 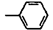 | 14.8 | | 30 | 80 |
| 87 | (CH₂)₃CH₃ | H | 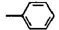 | 96.4 | | 10 | 50 |
| 88 | CH₂Ph | H | 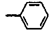 | 20.9 | | 10 | 30 |
| 89 | CH₂CH=CH₂ | H | 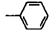 | 30.6 | | 10 | 80 |
| 90 | CH₂CH₂CH₃ | H | 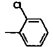 | 41.2 | | 10 | 50 |
| 91 | (CH₂)₃CH₃ | H | 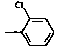 | 1.2 | | 0 | 80 |
| 92 | CH₂Ph | H | 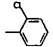 | 45.8 | | 50 | 70 |
| 93 | CH₂CH=CH₂ | H | 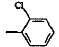 | 96.0 | | 40 | 70 |
| 94 | CH₂C≡CH | H | 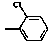 | 31.2 | | 50 | 60 |
| 95 | CH₃ | H | 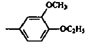 | 58.0 | | 40 | 80 |
| 96 | CH₂CH₂CH₃ | H | 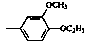 | 73.4 | | 20 | 50 |
| 97 | (CH₂)₃CH₃ | H | 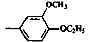 | 35.8 | | 40 | 70 |
| 98 | CH₂Ph | H | 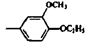 | 13.3 | | 40 | 10 |
| 99 | CH₂CH=CH₂ | H | 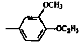 | 45.8 | | 50 | 30 |
| 100 | CH₂CH₂CH₃ | H | 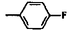 | 20.3 | | 20 | 90 |
| 101 | CH₂CH=CH₂ | H | 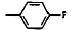 | 17.7 | | 40 | 30 |
| 102 | CH₂C≡CH | H | 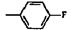 | 31.6 | | 50 | 50 |
| 103 | CH₂CH₂CH₃ | H | 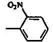 | 31.5 | | 20 | 60 |
| 104 | CH₂CH=CH₂ | H | 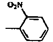 | / | | 40 | 50 |
| 105 | CH₂CH₂CH₃ | H | 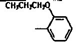 | 2.2 | | 50 | 50 |
| 106 | (CH₂)₃CH₃ | H | 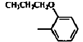 | 23.8 | | 30 | 30 |
| 107 | 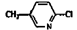 | H | 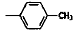 | 3.7 | | 10 | 20 |
| 108 | 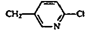 | H | 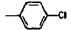 | / | | 50 | 50 |
| 109 | 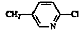 | H | 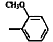 | / | | 40 | 90 |
| 110 | 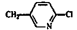 | H | 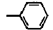 | 92.8 | 6.67 | 0 | 30 |
| 111 | 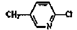 | H | 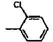 | 22.0 | 13.89 | 0 | 40 |
| 112 | 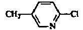 | H | 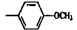 | 54.4 | 3.77 | 10 | 90 |
| 113 | 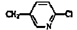 | H | 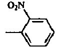 | 100 | 10.00 | 50 | 70 |
| 114 | 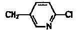 | H | 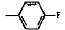 | 72.3 | 2.04 | 40 | 70 |
| 115 | 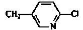 | H | 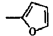 | 86.0 | 9.30 | 10 | 80 |
| 116 | CH₃ | H | 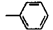 | | 10.29 | | |
| 117 | CH₃ | H | 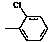 | | 17.78 | | |
| 118 | CH₃ | H | 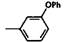 | | 14.81 | | |
| 119 | CH₃ | H | 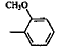 | | 72.00 | | |
| 120 | CH₃ | H | 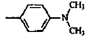 | | 82.76 | | |
| 121 | CH₃ | H | 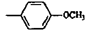 | | 16.95 | | |
| 122 | CH₃ | H | 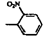 | | 21.82 | | |
| 123 | CH₃ | H | 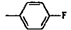 | | 4.69 | | |
| 124 | CH₃ | H | 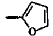 | | 88.68 | | |
| 125 | CH₂CH₃ | H | 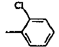 | | 53.06 | | |
| 126 | CH₂CH₃ | H | 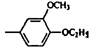 | | 6.67 | | |
| 127 | CH₂CH₃ | H | 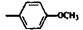 | | 24.56 | | |
| 128 | CH₂CH₃ | H | 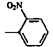 | | 19.05 | | |
| 129 | CH₂CH₃ | H | 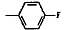 | | 8.62 | | |
| 130 | CH₂CH₂CH₃ | H | 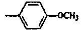 | | 18.46 | | |
| 131 | CH₂Ph | H | 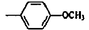 | | 1.89 | | |
| 132 | CH₂Ph | H | 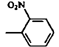 | | 9.68 | | |
| 133 | CH₂Ph | H | 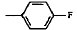 | | 3.77 | | |
| 134 | 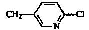 | H | 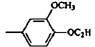 | | 1.82 | | |

**Table 2. The insecticidal activity of the compounds of formula I to Myzus persicae(24h).**

| Compound | Item | 400µg/mL | 2001µg/mL | 100µg/mL | 50µg/mL | 25µg/mL |
|---|---|---|---|---|---|---|
| 50 | numbers of death/total numbers | 96/96 | 103/107 | 87/87 | 91 /92 | 78/78 |
| | mortality(%) | 100 | 96.3 | 100 | 98.9 | 100 |
| 56 | numbers of death/total numbers | 77/77 | 81/81 | 149/149 | 96/99 | 104/104 |
| | mortality(%) | 100 | 100 | 100 | 97.0 | 100 |
| 87 | numbers of death/total numbers | 74/74 | 77/77 | 77/78 | 79/81 | 56/84 |
| | mortality(%) | 100 | 100 | 98.7 | 97.5 | 66.7 |
| 93 | numbers of death/total numbers | 93/93 | 114/114 | 104/104 | 94/98 | 112/112 |
| | mortality(%) | 100 | 100 | 100 | 95.9 | 100 |
| 113 | numbers of death/total numbers | 84/88 | 92/92 | 85/85 | 66/68 | 48/80 |
| | mortality(%) | 95.5 | 100 | 100 | 97.1 | 60.0 |
| CK | numbers of death/total numbers | 6/129 | | | | |
| | mortality(%) | 4.7 | | | | |

**Table 3. The insecticidal activity of the compounds of formula I to Hyalopterus pruni (24h).**

| Compound | Item | 400µg/mL | 200µg/mL | 100µg/mL | 50µg/mL | 25µg/mL |
|---|---|---|---|---|---|---|
| 50 | numbers of death/total numbers | 102/102 | 110/110 | 101/101 | 98/102 | 104/108 |
| | mortality(%) | 100 | 100 | 100 | 96.1 | 96.3 |
| 56 | numbers of death/total numbers | 103/103 | 101/101 | 121/121 | 96/101 | 100/104 |
| | mortality(%) | 100 | 100 | 100 | 95.0 | 96.2 |
| 87 | numbers of death/total numbers | 99/99 | 107/107 | 110/114 | 94/101 | 90/114 |
| | mortality(%) | 100 | 100 | 96.5 | 93.1 | 78.9 |
| 93 | numbers of death/total numbers | 103/103 | 110/110 | 103/103 | 104/108 | 102/112 |
| | mortality(%) | 100 | 100 | 100 | 96.3 | 91.1 |
| 113 | numbers of death/total numbers | 114/114 | 102/102 | 105/107 | 106/110 | 88/118 |
| | mortality(%) | 100 | 100 | 98.1 | 96.4 | 74.6 |
| CK | numbers of death/total numbers | 11/226 | | | | |
| | mortality(%) | 4.9 | | | | |

**Table 4. The insecticidal activity of the compounds of formula I to Aphis gossypii (24h).**

| compound | item | 50µg/mL | 12.5µg/mL | 3.13µg/mL |
|---|---|---|---|---|
| 50 | numbers of death/total numbers | 245/245 | 150/150 | 156/157 |
| | mortality(%) | 100 | 100 | 99.4 |
| 56 | numbers of death/total numbers | 166/178 | 136/164 | 121/157 |
| | morality(%) | 93.3 | 82.9 | 77.1 |
| 87 | numbers of death/total numbers | 99/104 | 99/114 | 141/185 |
| | mortality(%) | 95.2 | 86.8 | 76.2 |
| 93 | numbers of death/total numbers | 138/138 | 124/124 | 178/186 |
| | mortality(%) | 100 | 100 | 95.7 |
| 113 | numbers of death/total numbers | 176/176 | 132/132 | 104/121 |
| | mortality(%) | 100 | 100 | 86.0 |
| CK | numbers of death/total numbers | 1/85 | | |
| | mortality(%) | 1.2 | | |

### Industrial application

The present provides hydrocarbylidene nitrohydrozinecarboximidamides of structural general formula shown as formula I, and the use thereof as well as the method for making the same. These compounds were prepared by the inventors of the present invention through broadly investigation and rationally designing, as well as screening lots of compounds. Screened compounds have high insecticidal activity, and are prepared easily and conveniently. In addition, the present invention provides a preferred processing route, which has high safety and low cost, thus making practical value of these compounds be greatly improved.

The experiments of insecticidal activity show that the hydrocarbylidene nitrohydrozine -carboximidamides shown by formula (I) have high preventive efficiency against insect pests of plants, such as aphid, plant hopper, *Helicoverpa armigera*, *asparagus caterpillar*, and the like, so these compounds can be used as plant insecticides.

## Claims

1. Compounds shown by formula I or pharmaceutically acceptable salts thereof: 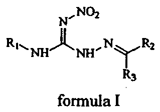 wherein :
R1 is C1 - C10 saturated and/or unsaturated aliphatic hydrocarbonyl, benzyl, substituted benzyl, halogenated picolyl, halogenated thiazolyl methyl, tetrahydrofuryl methyl or oxazolyl methyl;
R2 is hydrogen, C1 - C5 saturated and/or unsaturated aliphatic hydrocarbonyl, substituted phenyl, pyridyl or substituted pyridyl;
R3 is hydrogen, C1 - C10 saturated and/or unsaturated aliphatic hydrocarbonyl, furyl, phenyl, substituted phenyl, benzyl or substituted benzyl.

2. The compounds according to claim 1, **characterized in that** the R1 is C1 -C10 unsaturated aliphatic hydrocarbonyl, halogenated picolyl, halogenated thiazolyl methyl or tetrahydrofuryl methyl, wherein preferably R1 is allyl, propargyl or chloro-picolyl.

3. The compounds according to claim 1, **characterized in that** the R2 is hydrogen or C1 - C5 saturated and/or unsaturated aliphatic hydrocarbonyl, wherein preferably R2 is hydrogen.

4. The compounds according to any one of claims 1-3, **characterized in that** the R3 is substituted phenyl or C I - C10 saturated and/or unsaturated aliphatic hydrocarbonyl.

5. A method for making compounds shown by formula I: 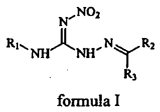 wherein :
R1 is C1 - C10 saturated and/or unsaturated aliphatic hydrocarbonyl, benzyl, substituted benzyl, halogenated picolyl, halogenated thiazolyl methyl, tetrahydrofuryl methyl or oxazolyl methyl;
R2 is hydrogen, C1 - C5 saturated and/or unsaturated aliphatic hydrocarbonyl, phenyl, substituted phenyl, pyridyl or substituted pyridyl;
R3 is hydrogen, C1 - C10 saturated and/or unsaturated aliphatic hydrocarbonyl. furyl, phenyl, substituted phenyl, benzyl or substituted benzyl,
the method comprises the following steps:
1) Reacting nitroguanidine with hydrazine hydrate to form the compounds shown by the structural general formula II; 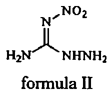
2) Reacting the compounds shown by the structural general formula II with the compounds shown by the structural general formula III under acid catalysis, to form the compounds shown by the structural general formula IV; 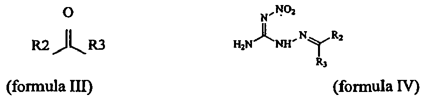
3) Reacting the compounds shown by the structural general formula IV with compounds shown by the structural general formula V under alkalis catalysis, to form the compounds shown by the structural general formula I;
R1-X (formula V)
wherein R2 of formula II and formula III are hydrogen, C1 - C5 saturated and/or unsaturated aliphatic hydrocarbonyl, phenyl, substituted phenyl, pyridyl or substituted pyridyl,
R3 is hydrogen, C1 - C10 saturated and/or unsaturated aliphatic hydrocarbonyl, furyl, phenyl, substituted phenyl, benzyl or substituted benzyl,
R1 of formula V is C1 - C10 saturated and/or unsaturated aliphatic hydrocarbonyl, benzyl, substituted benzyl, halogenated picolyl, halogenated thiazolyl methyl, tetrahydrofuryl methyl or oxazolyl methyl,
X of formula V is Cl, Br, I,

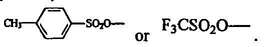

6. The method according to claim 5, **characterized in that** the reaction in step 1) is conducted in a solvent, and said solvent is water; and the reaction temperature of said reaction is 45-70°C; the molar ratio of nitroguanidine to hydrazine hydrate in step 1) is (1:1)-(1:1.5).

7. The method according to claim 5, **characterized in that** the reaction in step 2) is conducted in a solvent, and said solvent is anhydrous ethanol or methanol; the reaction temperature of said reaction is 50-80°C; the acids used in said reaction is acetic acid or p-toluenesulfonic acid; and the molar ratio of compounds shown by the structural general formula II to compounds shown by the structural general formula III in step 2) is (1:1)-(1:2).

8. The method according to claim 5, **characterized in that** the reaction in step 3) is conducted in a solvent, and said solvent is dimethylformamide or dimethylacetamide; the reaction temperature of said reaction is 0-50°C; the alkalis used in said reaction is sodium hydride, sodium ethoxide, sodium methoxide or sodium amide; and the molar ratio of the compounds shown by the structural general formula IV to the compounds shown by the structural general formula V in step 3) is (1: 1.2)-(1:2.5).

9. The use of compounds shown by formula I or pharmaceutically acceptable salts thereof or pharmaceutical compositions containing any one of them 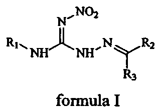 wherein :
R1 is C1 - C10 saturated and/or unsaturated aliphatic hydrocarbonyl, benzyl, substituted benzyl, halogenated picolyl, halogenated thiazolyl methyl, tetrahydrofuryl methyl or oxazolyl methyl;
R2 is hydrogen, C1 C5 saturated and/or unsaturated aliphatic hydrocarbonyl, phenyl, substituted phenyl, pyridyl or substituted pyridyl;
R3 is hydrogen, C1 - C10 saturated and/or unsaturated aliphatic hydrocarbonyl, furyl, phenyl, substituted phenyl, benzyl or substituted benzyl,
for manufacturing plant insecticides that kill homoptera pests and/or lepidoptera pests.

10. The use according to claim 9, **characterized in that** the homoptera pests are at least one family of the following six families: aphididae, aleyrodidae, delphacidae, psyllidae, jassidae and coccidae pests; and the lepidoptera pests are noctuidae and/or plutellidae.

11. A plant insecticidal drug or formulation whose active ingredient is a compound shown by formula I or pharmaceutically acceptable salts thereof 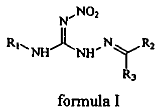 wherein :
R1 is C1 - C10 saturated and/or unsaturated aliphatic hydrocarbonyl, benzyl, substituted benzyl, halogenated pirolyl, halogenated thiazolyl methyl, tetrahydrofuryl methyl or oxazolyl methyl;
R2 is hydrogen, C1 - C5 saturated and/or unsaturated aliphatic hydrocarbonyl, phenyl, substituted phenyl, pyridyl or substituted pyridyl;
R3 is hydrogen, C1 C10 saturated and/or unsaturated aliphatic hydrocarbonyl, furyl, phenyl, substituted phenyl, benzyl or substituted benzyl,
wherein the active ingredient of the plant insecticidal drug or formulation is 0.01%-99.99% by mass.

12. The plant insecticidal drug or formulation according to claim 11, **characterized in that** the plant insecticidal drugs or formulations is the drug or formulation that kills homoptera pests and/or lepidoptera pests, including aphididae, aleyrodidae, delphacidae, psyllidae, jassidae and coccidae pests, noctuidae and/or plutellidae etc.

13. The use of the plant insecticidal drug or formulation according to any one of claims 11-12 in preventing insect pests of plants.

14. A method for preventing insect pests of plants by applying the plant insecticidal drug or formulation according to any one of claims 11-12 to plant leaves and/or plant fruits and/or plant seeds, and the places where the plant leaves and/or plant fruits and/or plant seeds are growing or are expected to be grown; the active ingredient of the plant insecticidal drug or formulation is administrated at the concentration of 1-600 mg/L.

15. The method according to claim 14, **characterized in that** the active ingredient of the plant insecticidal drug or formulation is administrated at the concentration of 3-50 mg/L.

## Patentansprüche

1. Verbindungen, gezeigt durch Formel 1 oder pharmazeutisch akzeptable Salze davon: 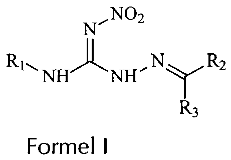 wobei:
R1 ein C1-C10 gesättigtes und/oder ungesättigtes aliphatisches Hydrocarbonyl, Benzyl, substituiertes Benzyl, halogeniertes Picolyl, halogeniertes Thiazolylmethyl, Tetrahydrofurylmethyl oder Oxazolylmethyl ist;
R2 Wasserstoff, ein C1-C5 gesättigtes und/oder ungesättigtes aliphatisches Hydrocarbonyl, substituiertes Phenyl, Pyridyl oder substituiertes Pyridyl ist;
R3 Wasserstoff, ein C1-C10 gesättigtes und/oder ungesättigtes aliphatisches Hydrocarbonyl, Furyl, Phenyl, substituiertes Phenyl, Benzyl oder substituiertes Benzyl ist.

2. Verbindungen nach Anspruch 1, **dadurch gekennzeichnet, dass** R1 ein C1-C10 ungesättigtes aliphatisches Hydrocarbonyl, halogeniertes Picolyl, halogeniertes Thiazolylmethyl oder Tetrahydrofurylmethyl ist, wobei R1 vorzugsweise Allyl, Propargyl oder Chloropicolyl ist.

3. Verbindungen nach Anspruch 1, **dadurch gekennzeichnet, dass** R2 Wasserstoff oder ein C1-C5 gesättigtes und/oder ungesättigtes aliphatisches Hydrocarbonyl ist, wobei R2 vorzugsweise Wasserstoff ist.

4. Verbindungen nach einem der Ansprüche 1-3, **dadurch gekennzeichnet, dass** R3 substituiertes Phenyl oder ein C1-C10 gesättigtes und/oder ungesättigtes aliphatisches Hydrocarbonyl ist.

5. Verfahren zur Herstellung von Verbindungen, wie gemäß Formel I gezeigt, 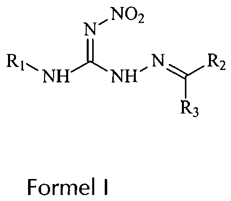 wobei R1 ein n C1-C10 gesättigtes und/oder ungesättigtes aliphatisches Hydrocarbonyl, Benzyl, substituiertes Benzyl, halogeniertes Picolyl, halogeniertes Thiazolylmethyl, Tetrahydrofurylmethyl oder Oxazolylmethyl ist;
R2 Wasserstoff, ein C1-C5 gesättigtes und/oder ungesättigtes aliphatisches Hydrocarbonyl, Phenyl, substituiertes Phenyl, Pyridyl oder substituiertes Pyridyl ist;
R3 Wasserstoff, ein C1-C10 gesättigtes und/oder ungesättigtes aliphatisches Hydrocarbonyl, Furyl, Phenyl, substituiertes Phenyl, Benzyl oder substituiertes Benzyl ist, wobei das Verfahren die folgenden Schritte umfasst:
(1) Reaktion von Nitroguanidin mit Hydrazinhydrat zur Bildung von Verbindungen, wie durch die allgemeine Strukturformel II gezeigt: 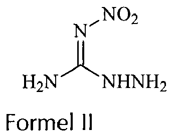
(2) Reaktion von Verbindungen, wie durch die allgemeine Strukturformel II gezeigt, mit den Verbindungen, wie durch die allgemeine Strukturformel III gezeigt, bei saurer Katalyse zur Bildung von Verbindungen, wie durch die allgemeine Strukturformel IV gezeigt: 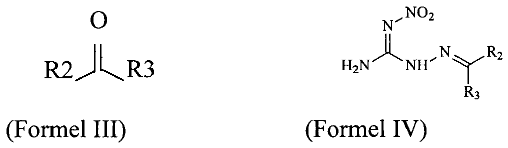
(3) Reaktion der Verbindungen, wie durch die allgemeine Strukturformel IV gezeigt, mit Verbindungen, wie durch die allgemeine Strukturformel V gezeigt, bei alkalischer Katalyse zur Bildung von Verbindungen, wie durch die allgemeine Strukturformel V gezeigt
RI-X (Formel V)
wobei
R2 der Formel II und der Formel III Wasserstoff, ein C1-C5 gesättigtes und/oder ungesättigtes aliphatisches Hydrocarbonyl, Phenyl, substituiertes Phenyl, Pyridyl oder substituiertes Pyridyl ist,
R3 Wasserstoff, ein C1-C10 gesättigtes und/oder ungesättigtes aliphatisches Hydrocarbonyl, Furyl, Phenyl, substituiertes Phenyl, Benzyl oder substituiertes Benzyl ist,
R1 der Formel V ein C1-C10 gesättigtes und/oder ungesättigtes aliphatisches Hydrocarbonyl, Benzyl, substituiertes Benzyl, halogeniertes Picolyl, halogeniertes Thiazolylmethyl, Tetrahydrofurylmethyl oder Oxazolylmethyl ist,
X der Formel V
CI,Br,I, 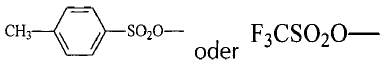 ist.

6. Verfahren nach Anspruch 5, **dadurch gekennzeichnet, dass** der Reaktionsschritt 1) in einem Lösungsmittel ausgeführt wird und dieses Lösungsmittel Wasser ist; und die Reaktionstemperatur dieser Reaktion zwischen 45 und 70°C ist; wobei das molare Verhältnis von Nitroguanidin zu Hydrazinhydrat in Schritt 1) (1:1)-(1:1,5) ist.

7. Verfahren nach Anspruch 5, **dadurch gekennzeichnet, dass** der Reaktionsschritt 2) in einem Lösungsmittel ausgeführt wird und dieses Lösungsmittel wasserfreies Ethanol oder Methanol ist; die Reaktionstemperatur dieser Reaktion 50 bis 80°C beträgt; die verwendeten Säuren in dieser Reaktion Essigsäure oder p-Toluolsulfonsäure ist; und das molare Verhältnis der Verbindungen, wie durch die allgemeine Strukturformel II gezeigt, zu Verbindungen, wie durch die allgemeine Strukturformel III gezeigt, in Schritt 2) (1:1)-(1:2) ist.

8. Verfahren nach Anspruch 5, **dadurch gekennzeichnet, dass** die Reaktion in Schritt 3) in einem Lösungsmittel ausgeführt wird und dieses Lösungsmittel Dimethylformamid oder Dimethylacetamid ist; die Reaktionstemperatur dieser Reaktion 0 bis 50°C ist; die alkalischen Bestandteile, die in dieser Reaktion verwendet werden, Natriumhydrid, Natriumethoxid, Natriummethoxid oder Natriumamid sind; und das molare Verhältnis der Verbindungen, wie durch die allgemeine Strukturformel IV gezeigt, zu den Verbindungen, wie durch die allgemeine Strukturformel V gezeigt, in Schritt 3) (1:1,2)-(1:2,5) ist.

9. Verwendung von Verbindungen, wie durch die Formel I gezeigt oder pharmazeutisch akzeptablen Salzen davon oder pharmazeutischen Zusammensetzungen, die eine von diesen enthalten: 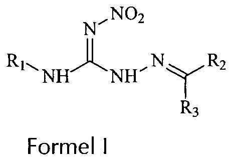 wobei:
R1 ein C1-C10 gesättigtes und/oder ungesättigtes aliphatisches Hydrocarbonyl, Benzyl, substituiertes Benzyl, halogeniertes Picolyl, halogeniertes Thiazolylmethyl, Tetrahydrofurylmethyl oder Oxazolylmethyl ist;
R2 Wasserstoff, ein C1-C5 gesättigtes und/oder ungesättigtes aliphatisches Hydrocarbonyl, Phenyl, substituiertes Phenyl, Pyridyl oder substituiertes Pyridyl ist;
R3 Wasserstoff, ein C1-C10 gesättigtes und/oder ungesättigtes aliphatisches Hydrocarbonyl, Furyl, Phenyl, substituiertes Phenyl, Benzyl oder substituiertes Benzyl ist,
zur Herstellung von Insektiziden für Pflanzen, die die homopteren und/oder lepidopteren Schädlinge abtöten.

10. Verwendung nach Anspruch 9, **dadurch gekennzeichnet, dass** die homopteren Schädlinge wenigstens eine der folgenden sechs Familien sind: Aphididae-, Aleyrodidae-, Delphacidae-, Psyllidae-, Jassidae- und Coccidae-Pesterkrankungen, und die lepidopteren Schädlinge Noctuidae und/oder Plutellidae sind.

11. Insektizider Wirkstoff oder Formulierung für Pflanzen, dessen/deren aktiver Inhaltsstoff eine Verbindung, wie durch Formel I gezeigt oder pharmazeutisch akzeptable Salze davon ist/sind: 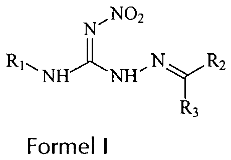 wobei:
R1 ein C1-C10 gesättigtes und/oder ungesättigtes aliphatisches Hydrocarbonyl, Benzyl, substituiertes Benzyl, halogeniertes Picolyl, halogeniertes Thiazolylmethyl, Tetrahydrofurylmethyl oder Oxazolylmethyl ist;
R2 Wasserstoff, ein C1-C5 gesättigtes und/oder ungesättigtes aliphatisches Hydrocarbonyl, Phenyl, substituiertes Phenyl, Pyridyl oder substituiertes Pyridyl ist;
R3 Wasserstoff, ein C1-C10 gesättigtes und/oder ungesättigtes aliphatisches Hydrocarbonyl, Furyl, Phenyl, substituiertes Phenyl, Benzyl oder substituiertes Benzyl ist,
wobei der aktive Inhaltsstoff des insektiziden Wirkstoffs oder der Formulierung für Pflanzen 0,01 Massen%-99,99 Massen% beträgt.

12. lnsektizider Wirkstoff oder Formulierung für Pflanzen nach Anspruch 11, **dadurch gekennzeichnet, dass** die insektiziden Wirkstoffe oder Formulierungen für Pflanzen der Wirkstoff oder die Formulierung is/sind, die homoptere und/oder lepidoptere Schädlinge abtöten, einschließlich Aphididae-, Aleyrodidae-, Delphacidae-, Psyllidae-, Jassidae- und Coccidae-Pesterkrankungen, Noctuidae und/oder Plutellidae etc.

13. Verwendung des insektiziden Wirkstoffs oder der Formulierung für Pflanzen nach einem der Ansprüche 11 oder 12 zur Verhinderung von pflanzlichen Insekten-Schädlingen.

14. Verfahren zur Verhinderung von Insekten-Schädlingen von Pflanzen durch Anwendung des insektiziden Wirkstoffs oder der Formulierung für Pflanzen nach einem der Ansprüche 11 oder 12 auf Pflanzenblätter und/oder Pflanzenfrüchte und/oder Pflanzensamen, und an Bereiche, wo die Pflanzenblätter und/oder Pflanzenfrüchte und/oder Pflanzensamen wachsen oder deren Wachstum erwartet wird; wobei der aktive Inhaltsstoff des insektiziden Wirkstoffs oder der Formulierung für Pflanzen bei einer Konzentration von 1-600 mg/l verabreicht wird.

15. Verfahren nach Anspruch 14, **dadurch gekennzeichnet, dass** der aktive Inhaltsstoff des insektiziden Wirkstoffs oder der Formulierung für Pflanzen bei einer Konzentration von 3-50 mg/l verabreicht wird.

## Revendications

1. Composés représentés par la formule I ou sels pharmaceutiquement acceptables de ceux-ci : 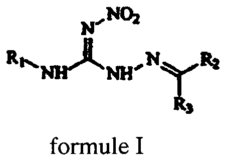 où :
R1 est C1 - C10 hydrocarbonyle aliphatique saturé et/ou insaturé, benzyle, benzyle substitué, picolyle halogéné, thiazolylméthyle halogéné, tétrahydrofurylméthyle ou oxazolylméthyle ;
R2 est l'hydrogène, C1 - C5 hydrocarbonyle aliphatique saturé et/ou insaturé, phényle substitué, pyridyle ou pyridyle substitué ;
R3 est l'hydrogène, C1 - C10 hydrocarbonyle aliphatique saturé et/ou insaturé, furyle, phényle, phényle substitué, benzyle ou benzyle substitué.

2. Composés selon la revendication 1 **caractérisés en ce que** le R1 est C1 - C10 hydrocarbonyle aliphatique insaturé, picolyle halogéné, thiazolylméthyle halogéné ou tétrahydrofurylméthyle, où de préférence R1 est allyle, propargyle ou chloropicolyle.

3. Composés selon la revendication 1 **caractérisés en ce que** le R2 est l'hydrogène ou C1 - C5 hydrocarbonyle aliphatique saturé et/ou insaturé, où de préférence R2 est l'hydrogène.

4. Composés selon l'une quelconque des revendications 1-3 **caractérisés en ce que** R3 est phényle substitué ou C1 - C10 hydrocarbonyle aliphatique saturé et/ou insaturé.

5. Procédé pour produire des composés représentés par la formule I : 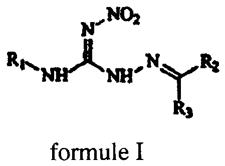 où :
R1 est C1 - C10 hydrocarbonyle aliphatique saturé et/ou insaturé, benzyle, benzyle substitué, picolyle halogéné, thiazolylméthyle halogéné, tétrahydrofurylméthyle ou oxazolylméthyle ;
R2 est l'hydrogène, C1 - C5 hydrocarbonyle aliphatique saturé et/ou insaturé, phényle, phényle substitué, pyridyle ou pyridyle substitué ;
R3 est l'hydrogène, C1 - C10 hydrocarbonyle aliphatique saturé et/ou insaturé, furyle, phényle, phényle substitué, benzyle ou benzyle substitué,
le procédé comprend les étapes suivantes :
1) réaction de la nitroguanidine avec l'hydrate d'hydrazine pour former les composés représentés par la formule générale structurale II ; 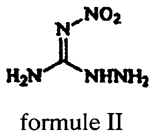
2) réaction des composés représentés par la formule générale structurale II avec les composés représentés par la formule générale structurale III sous catalyse acide, pour former les composés représentés par la formule générale structurale IV ; 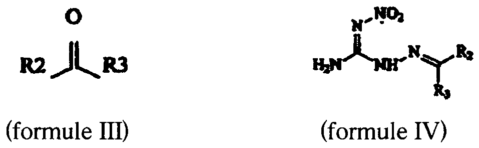
3) réaction des composés représentés par la formule générale structurale IV avec des composés représentés par la formule générale structurale V sous catalyse alcaline, pour former les composés représentés par la formule générale structurale 1 ;
R1-X (formule V)
où les R2 de la formule II et de la formule III sont l'hydrogène, C1 - C5 hydrocarbonyle aliphatique saturé et/ou insaturé, phényle, phényle substitué, pyridyle ou pyridyle substitué,
R3 est l'hydrogène, C1 - C10 hydrocarbonyle aliphatique saturé et/ou insaturé, furyle, phényle, phényle substitué, benzyle ou benzyle substitué,
R1 de la formule V est C1 - C10 hydrocarbonyle aliphatique saturé et/ou insaturé, benzyle, benzyle substitué, picolyle halogéné, thiazolylméthyle halogéné, tétrahydrofurylméthyle ou oxazolylméthyle ;
X de la formule V est CI, Br, I, 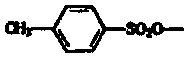 ou F₃CSO₂O-.

6. Procédé selon la revendication 5 **caractérisé en ce que** la réaction dans l'étape 1) est conduite dans un solvant, et ledit solvant est l'eau ; et la température de réaction de ladite réaction est 45-70°C ; le rapport molaire de la nitroguanidine à l'hydrate d'hydrazine dans l'étape 1) est (1:1)-(1:1,5).

7. Procédé selon la revendication 5 **caractérisé en ce que** la réaction dans l'étape 2) est conduite dans un solvant, et ledit solvant est l'éthanol ou le méthanol anhydre ; la température de réaction de ladite réaction est 50-80°C ; les acides utilisés dans ladite réaction sont l'acide acétique ou l'acide p-toluènesulfonique ; et le rapport molaire des composés représentés par la formule générale structurale II aux composés représentés par la formule générale structurale III dans l'étape 2) est (1:1)-(1:2).

8. Procédé selon la revendication 5 **caractérisé en ce que** la réaction dans l'étape 3) est conduite dans un solvant, et ledit solvant est le diméthylformamide ou le diméthylacétamide ; la température de réaction de ladite réaction est 0-50°C ; les alcalis utilisés dans ladite réaction sont l'hydrure de sodium, l'éthylate de sodium, le méthylate de sodium ou l'amidure de sodium ; et le rapport molaire des composés représentés par la formule générale structurale IV aux composés représentés par la formule générale structurale V dans l'étape 3) est (1:1,2)-(1:2,5).

9. Utilisation de composés représentés par la formule I ou de sels pharmaceutiquement acceptables de ceux-ci ou de compositions pharmaceutiques contenant l'un quelconque de ceux-ci : 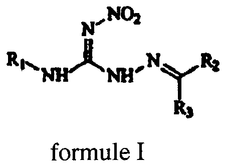 où :
R1 est C1 - C10 hydrocarbonyle aliphatique saturé et/ou insaturé, benzyle, benzyle substitué, picolyle halogéné, thiazolylméthyle halogéné, tétrahydrofurylméthyle ou oxazolylméthyle ;
R2 est l'hydrogène, C1 - C5 hydrocarbonyle aliphatique saturé et/ou insaturé, phényle, phényle substitué, pyridyle ou pyridyle substitué ;
R3 est l'hydrogène, C1 - C10 hydrocarbonyle aliphatique saturé et/ou insaturé, furyle, phényle, phényle substitué, benzyle ou benzyle substitué,
pour fabriquer des insecticides pour les plantes qui tuent les nuisibles homoptères et/ou les nuisibles lépidoptères.

10. Utilisation selon la revendication 9 **caractérisée en ce que** les nuisibles homoptères sont au moins une famille des six familles suivantes : nuisibles aphididés, aleyrodidés, delphacidés, psyllidés, jassidés et coccidés ; et les nuisibles lépidoptères sont des noctuidés et/ou des plutellidés.

11. Médicament ou formulation insecticide pour les plantes dont l'ingrédient actif est un composé représenté par la formule I ou des sels pharmaceutiquement acceptables de celui-ci : 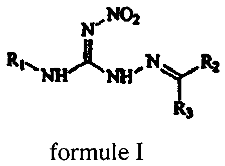 où :
R1 est C1 - C10 hydrocarbonyle aliphatique saturé et/ou insaturé, benzyle, benzyle substitué, picolyle halogéné, thiazolylméthyle halogéné, tétrahydrofurylméthyle ou oxazolylméthyle ;
R2 est l'hydrogène, C1 - C5 hydrocarbonyle aliphatique saturé et/ou insaturé, phényle, phényle substitué, pyridyle ou pyridyle substitué ;
R3 est l'hydrogène, C1 - C10 hydrocarbonyle aliphatique saturé et/ou insaturé, furyle, phényle, phényle substitué, benzyle ou benzyle substitué,
où l'ingrédient actif du médicament ou formulation insecticide pour les plantes est 0,01%-99,99% en masse.

12. Médicament ou formulation insecticide pour les plantes selon la revendication 11 **caractérisé en ce que** les médicaments ou formulations insecticides pour les plantes sont le médicament ou formulation qui tue les nuisibles homoptères et/ou les nuisibles lépidoptères, incluant les nuisibles aphididés, aleyrodidés, delphacidés, psyllidés, jassidés et coccidés, les noctuidés et/ou plutellidés, etc.

13. Utilisation du médicament ou formulation insecticide pour les plantes selon l'une quelconque des revendications 11-12 dans la prévention des insectes nuisibles des plantes.

14. Procédé de prévention des insectes nuisibles des plantes par application du médicament ou formulation insecticide pour les plantes selon l'une quelconque des revendications 11-12 à des feuilles de plantes et/ou des fruits de plantes et/ou des graines de plantes, et aux endroits où les feuilles de plantes et/ou les fruits de plantes et/ou les graines de plantes poussent ou sont suspectés de pousser ; l'ingrédient actif du médicament ou formulation insecticide pour les plantes est administré à la concentration de 1-600 mg/L.

15. Procédé selon la revendication 14 **caractérisé en ce que** l'ingrédient actif du médicament ou formulation insecticide pour les plantes est administré à la concentration de 3-50 mg/L.
